(19) Europäisches Patentamt
European Patent Office
Office européen des brevets

(11) Veröffentlichungsnummer : **0 088 961 B1**

# EUROPÄISCHE PATENTSCHRIFT ⑫

(45) Veröffentlichungstag der Patentschrift :
28.11.84

(21) Anmeldenummer : 83102126.6

(22) Anmeldetag : 04.03.83

(51) Int. Cl.³ : **C 07 C 51/27, C 07 C 59/125, C 07 C 51/487**

(54) **Verfahren zur Herstellung von 2-Methoxyalkansäuren.**

(30) Priorität : 17.03.82 DE 3209622

(43) Veröffentlichungstag der Anmeldung :
21.09.83 Patentblatt 83/38

(45) Bekanntmachung des Hinweises auf die Patenterteilung : 28.11.84 Patentblatt 84/48

(84) Benannte Vertragsstaaten :
CH DE FR LI SE

(56) Entgegenhaltungen :
EP-A- 0 048 396
DE-A- 2 832 949
HOUBEN-WEYL: "Methoden der Organischen Chemie", 4. Auflage, Band IV/1a, "Oxidation, Teil I", 1981, Seiten 649-656, Georg Thieme Verlag, Stuttgart, DE.

(73) Patentinhaber : **BASF Aktiengesellschaft**
**Carl-Bosch-Strasse 38**
**D-6700 Ludwigshafen (DE)**

(72) Erfinder : **Disteldorf, Walter, Dr.**
**Portugieser Weg 17**
**D-6706 Wachenheim (DE)**
Erfinder : **Elsfeld, Wolfgang, Dr.**
**Dubliner Strasse 6**
**D-6700 Ludwigshafen (DE)**
Erfinder : **Hellbach, Hans**
**Stettiner Strasse 14**
**D-6840 Lampertheim (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von 2-Methoxyalkansäuren mit 2 bis 5 C-Atomen durch Oxidation der entsprechenden 2-Methoxyalkanole mit Salpetersäure.

In der DE-OS 28 32 949 wird ein Verfahren zur Herstellung von Methoxyessigsäure beschrieben, bei dem man 2-Methoxyethanol in Gegenwart eines Vanadiumkatalysators mit Salpetersäure oxidiert. Bei diesem bekannten Verfahren, bei dem man 3 bis 5 Mol Salpetersäure je Mol 2-Methoxyethanol anwendet, erwärmt man die Salpetersäure mit einem Teil des 2-Methoxyethanols zur Induzierung der Reaktion kurzfristig auf 90 °C, wonach die Oxidation bei 40 bis 60 °C zu Ende geführt wird. Vor Isolierung der Methoxyessigsäure wird der größte Teil der nicht umgesetzten Salpetersäure im Vakuum abdestilliert. Dann gibt man Formaldehyd oder Paraformaldehyd hinzu und reduziert anschließend die noch vorhandene salpetrige Säure bzw. Stickoxide durch Zugabe von Harnstoff.

Dieses Verfahren bietet bei der technischen Durchführung Risiken. So geht bei Beginn der Reaktion die Oxidation bei einer Temperatur von etwa 90 °C über die Stufe der Methoxyessigsäure hinaus. Andestillieren nach der Oxidation unter Vakuum führt zunächst zu einer vollständigen Entwässerung des Reaktionsgemisches und damit zu einer Anreicherung von hochkonzentrierter Salpetersäure im Sumpf, wodurch detonationsfähige Gemische entstehen. Versucht man, die salpetersauren Oxidationsgemische drucklos zu destillieren, so kann man zwar bis zu einer Sumpftemperatur von 150 °C die Salpetersäure gefahrlos entfernen, hat hierbei jedoch hohe Ausbeuteverluste durch Überoxidation hinzunehmen. Außerdem weist die so erhaltene Rohsäure einen N-Gehalt von etwa 0,3 % auf.

Es wurde nun gefunden, daß man 2-Methoxyalkansäuren mit 2 bis 5 C-Atomen durch Oxidation der entsprechenden 2-Methoxyalkanole mit Salpetersäure in Gegenwart von Vanadationen als Katalysator bei Temperaturen unter 90 °C und destillative Abtrennung der 2-Methoxyalkansäure mit hoher Selektivität und technischer Sicherheit herstellen kann, wenn man

a) weniger als 3 Mol Salpetersäure, bezogen auf 1 Mol 2-Methoxyalkanol, verwendet,

b) die Oxidation bei Temperaturen von 40 bis 80 °C durchführt,

c) das Reaktionsgemisch bei 40 bis 100 °C mit einem Überschuß an Methoxyacetaldehyd oder 2-Methoxypropionaldehyd behandelt und dann unter Abdestillieren von Wasser ohne Vakuum auf Temperaturen von über 100 °C erhitzt und

d) den Rückstand nach Isolierung der 2-Methoxyalkansäure durch fraktionierte Destillation erneut als Katalysator für die Oxidation von 2-Methoxyalkanol nach diesem Verfahren einsetzt.

Nach dem neuen Verfahren werden 2-Methoxyalkanole mit 2 bis 5 C-Atomen, wie 2-Methoxyethanol oder 2-Methoxypropanol, mit weniger als 3 Mol, vorzugsweise 2,5 bis 1,8 Mol Salpetersäure, bezogen auf ein Mol Methoxyalkanol oxidiert.

Als Salpetersäure verwendet man zweckmäßigerweise 30 bis 80 %ige wäßrige Salpetersäure, die bei der diskontinuierlichen Arbeitsweise vorzugsweise insgesamt vorgelegt wird.

Die Oxidation führt man bei Temperaturen von 40 bis 80 °C durch. Zum verzögerungsfreien Starten der Oxidation hat es sich als vorteilhaft erwiesen, das zum Einleiten der Reaktion benötigte freie $NO_2$ durch Zugabe von Methoxyacetaldehyd, 2-Methoxypropionaldehyd, Acetaldehyd, Paraldehyd, Metaldehyd oder Propionaldehyd bei Temperaturen von 40 bis 60 °C zur vorgelegten Salpetersäure zu erzeugen. Von den genannten Aldehyden sind Methoxyacetaldehyd und 2-Methoxypropionaldehyd bevorzugt. Da die Menge an freiem $NO_2$ 0,1 bis 2, vorzugsweise 0,3 bis 0,9 Gew.%, bezogen auf die wäßrige Salpetersäure, betragen sollte, gibt man, bezogen auf die wäßrige Salpetersäure, 0,05 bis 2,5, vorzugsweise 0,2 bis 1 Gew.% des Aldehyds zur vorgelegten Salpetersäure, wobei man zweckmäßig den Aldehyd einsetzt, der der gewünschten 2-Methoxyalkansäure zugrunde liegt.

Als Katalysatoren verwendet man Vanadationen enthaltende Katalysatoren. Man gibt sie, z. B. in Form von Ammoniumvanadat, Vanadinpentoxid oder Vanadinoxidsulfat zur vorgelegten Salpetersäure. Besonders vorteilhafte Ergebnisse werden erzielt, wenn man Katalysatoren verwendet, die Vanadat- und Kupfersalzen, wie Kupferacetat, -carbonat oder -nitrat oder als metallisches Kupfer zugegeben. Der Katalysator wird z. B. in einer Menge von 0,003 bis 0,3, vorzugsweise 0,03 bis 0,07 Gew.% (berechnet auf V und Cu), bezogen auf die wäßrige Salpetersäure, eingesetzt. Das g-Atomverhältnis Vanadium zu Kupfer kann z. B. 5 : 1 bis 1 : 8 betragen. Besonders günstig hat sich ein g-Atomverhältnis von 1 : 1 bis 1 : 4 erwiesen.

Die Oxidation selbst wird in einem Temperaturbereich von 40 bis 80 °C drucklos durchgeführt, wobei man bis zum 85 %igen Umsatz eine Temperatur von 60 °C nicht überschreiten sollte. Zur Nachoxidation kann dann die Temperatur auf 80 °C erhöht werden.

Nach der Erfindung wird das Reaktionsgemisch dadurch aufgearbeitet, daß man zum Abbau restlicher Salpetersäure einen Überschuß an Methoxyacetaldehyd oder 2-Methoxypropionaldehyd hinzugibt. Die erforderlichen Mengen sind pro Mol noch vorhandener Salpetersäure 0,8 bis 2,4, vorzugsweise 1,0 bis 1,3 Mol Aldehyd. Der Aldehyd wird dabei zweckmäßigerweise als wäßrige, z. B. 90 %ige Lösung eingesetzt. Diese Behandlung wird bei Temperaturen von 40 bis 100 °C, vorzugsweise 65 bis 75 °C durchgeführt. Die Behandlungsdauer beträgt mindestens 30 Minuten, vorzugsweise etwa 1 bis 3 Stunden. Nach dieser Behandlung ist der Aldehyd unter weitgehender Zerstörung der Salpetersäure zum größten Teil in die gewünschte Säure überführt worden. Nun wird unter gleichzeitigem Abdestillieren von Wasser ohne Vakuum auf Temperaturen über 100 °C, beispielsweise auf 100 bis 120 °C, erhitzt, wobei man als Rückstand ein Konzentrat mit hohem Gehalt an 2-Methoxyalkansäure erhält, das überraschenderweise einen Salpetersäuregehalt von unter 0,01 Gew.% aufweist. Bei Verwendung eines Vanadationen oder Kupfer- und Vanadationen enthaltenden Katalysators ist diese weitgehende Zerstörung der Salpetersäure an einem Farbumschlag von blaugrün nach tiefblau zu erkennen, der durch Reduktion des Vanadats zum $VO^{2+}$-Ion bedingt ist.

Zur Isolierung der 2-Methoxyalkansäure wird der flüssige Rückstand aus der vorangegangenen Aufkonzentrierung fraktioniert destilliert, vorzugsweise im Vakuum. Im Destillationssumpf verbleibt die gesamte eingesetzte Vanadium- und Kupfermenge. Ohne weitere Behandlung wird der Sumpf in Salpetersäure aufgenommen und ohne Zugabe eines Katalysators für eine erneute Oxidation eingesetzt. Selbst nach 10-maliger Recyclisierung ist weder ein Ausbeuteverlust an 2-Methoxyalkansäure noch eine Gewichtszunahme beim Destillationssumpf festzustellen. Der N-Gehalt des Sumpfes liegt stets < 0,1 % und bildet damit kein Sicherheitsrisiko.

Man kann das neue Verfahren sowohl diskontinuierlich als auch kontinuierlich durchführen. Hierbei richtet sich die Dosierrate des 2-Methoxyalkanols nach den Möglichkeiten der Wärmeabfuhr. Durch den Einsatz von weniger als 3 Mol Salpetersäure pro Mol 2-Methoxyalkanol erhält man nach der 2-Methoxyalkanol-Zugabe zunächst eine Oxidationslösung, die bis zu 15 % des angesetzten Methylglykols in Form von 2-Methoxyalkyl-2-methoxyalkanat enthält. Dieser Ester wird jedoch unter den Reaktionsbedingungen bei Temperaturen von 60 bis 80 °C verseift, wobei das freigesetzte 2-Methoxyalkanol zur gewünschten Säure aufoxidiert wird. Zu diesem Zeitpunkt ist die Salpetersäure-Konzentration in der Oxidationslösung soweit vermindert, daß eine merkliche saure Etherspaltung, selbst bei Temperaturanhebung bis auf 80 °C, unterbleibt.

Nach dem erfindungsgemäßen Verfahren werden die 2-Methoxyalkansäuren in hoher Ausbeute völlig gefahrlos und technisch besonders einfach erhalten. Darüber hinaus bietet die beliebige Wiederverwendung des Sumpfes aus der fraktionierten Destillation als Katalysator für neue Ansätze einen unerwarteten Vorteil, besonders auch im Hinblick auf die damit erheblich verminderte Umweltbelastung.

Die 2-Methoxyalkansäuren, insbesondere die Methoxyessigsäure, sind z. B. als Hilfsmittel in der Textilindustrie oder als Zwischenprodukte für die Herstellung von Fungiziden anwendbar.

Beispiel 1

In einem Rührbehälter aus salpetersäurebeständigem Material, der mit Heiz- und Kühlvorrichtung, Rückflußkühler und Abgasleitung zur Nitrosevernichtung versehen ist, werden 3 500 Teile 63 %ige Salpetersäure, 1,75 Teile $NH_4VO_3$ und 3,85 Teile $Cu(Ac)_2 \cdot H_2O$ vorgelegt. Das g-Atomverhältnis Vanadium zu Kupfer beträgt in diesem Fall 1 bis 1,6. Man erwärmt auf 55 °C und gibt unter Rühren 15,5 Teile einer 90 %igen wäßrigen Methoxyacetaldehyd-Lösung (das sind 0,4 Gew.% Methoxyacetaldehyd, bezogen auf die 63 %ige Salpetersäure) hinzu, wobei sich die Lösung durch $NO_2$-Bildung dunkelbraun färbt. Über einen Zeitraum von 4 bis 6 Stunden werden 1 064 Teile 2-Methoxyethanol so zugegeben, daß eine Temperatur von 50 bis 55 °C eingehalten wird. Das Molverhältnis Salpetersäure zu 2-Methoxyethanol beträgt in diesem Beispiel 2,5 bis 1. Das Reaktionsgemisch wird nach der Zugabe des Methoxyethanols noch 1 Stunde bei 60 °C gehalten. Wie sich durch GC-Analyse feststellen läßt, sind 85 % des 2-Methoxyethanols oxidiert worden. Man oxidiert nach, indem man das Gemisch 3 Stunden auf 80 °C hält. Zur Zerstörung der Salpetersäure werden nun bei 80 bis 75 °C innerhalb von 1 bis 2 Stunden 672 Teile einer wäßrigen 90 %igen Methoxyacetaldehyd-Lösung (das sind 1,1 Mol Methoxyacetaldehyd, bezogen auf 1 Mol Salpetersäure im Oxidationsaustrag) gleichmäßig zugegeben. Danach wird noch 1 Stunde bei 90 °C gerührt. Dann wird zunächst bei Normaldruck bis zu einer Sumpftemperatur von 120 °C Wasser abdestilliert, wobei man als Sumpf eine dunkelblaue Lösung erhält, die < 0,01 Gew.% Salpetersäure, < 0,1 Gew.% Glykolsäure und < 0,1 Gew.% Oxalsäure enthält. Schließlich wird bei 100 mbar fraktionierend destilliert. Es werden 1 645 Teile Methoxyessigsäure mit einem Wassergehalt von unter 0,2 Gew.% erhalten. Die Ausbeute beträgt 88,1 Mol.%, bezogen auf den Alkohol und 70,5 Mol.%, bezogen auf den Aldehyd. Der den Katalysator enthaltende Destillationssumpf (14 bis 17 Teile) wird ohne Aufarbeitung für einen erneuten Einsatz gemäß diesem Beispiel verwendet, wobei auch nach 10 maliger Recyclisierung gütegleiche Methoxyessigsäure in gleicher Ausbeute und eine etwa gleichbleibende Destillationssumpfmenge gewonnen werden.

Beispiel 2

Man verfährt wie im Beispiel 1 beschrieben, wobei man jedoch anstelle von 1 064 Teilen 2-Methoxyethanol 1 260 Teile 2-Methoxypropanol und für das Starten anstelle von 14 Teile Methoxyacetaldehyd 19 Teile einer 90 %igen wäßrigen Lösung von 2-Methoxypropionaldehyd und für die Zerstörung der Salpetersäure 799 Teile einer 90 %igen Lösung von 2-Methoxypropionaldehyd einsetzt. Man erhält 1 737 Teile 2-Methoxypropionsäure mit einem Wassergehalt von unter 0,2 Gew.% ; Ausbeute 77,8 Mol.%, bezogen auf den Alkohol und 69,3 Mol.%, bezogen auf den Aldehyd. Der den Katalysator enthaltende Destillationssumpf ist gemäß Beispiel 1 rückführbar.

## Ansprüche

1. Verfahren zur Herstellung von 2-Methoxyalkansäuren mit 2 bis 5 C-Atomen durch Oxidation der entsprechenden 2-Methoxyalkanole mit Salpetersäure in Gegenwart von Vanadationen als Katalysator bei Temperaturen unter 90 °C und destillative Abtrennung der 2-Methoxyalkansäure, dadurch gekennzeichnet, daß man

a) weniger als 3 Mol Salpetersäure, bezogen auf 1 Mol Methoxyalkanol, verwendet,

b) die Oxidation bei Temperaturen von 40 bis 80 °C durchführt,

c) das Reaktionsgemisch bei 40 bis 100 °C mit einem Überschuß an Methoxyacetaldehyd oder 2-Methoxypropionaldehyd behandelt und dann unter Abdestillieren von Wasser ohne Vakuum auf Temperaturen von über 100 °C erhitzt und

d) den Rückstand nach Isolierung der 2-Methoxyalkansäure durch fraktionierte Destillation erneut als Katalysator für die Oxidation von 2-Methoxyalkanol nach diesem Verfahren einsetzt.

2. Verfahren nach Anspruch 1, dadurch gekennzeichnet, daß man 2,5 bis 1,8 Mol Salpetersäure pro Mol 2-Methoxyalkanol anwendet.

3. Verfahren nach Anspruch 1, dadurch gekennzeichnet, daß man die Salpetersäure und den Katalysator vorlegt und vor der Zugabe des 2-Methoxyalkanols 0,05 bis 2,5 Gew.%, bezogen auf die wäßrige Salpetersäure an Methoxyacetaldehyd, 2-Methoxypropionaldehyd, Acetaldehyd, Paraldehyd, Metaldehyd oder Propionaldehyd bei Temperaturen von 40 bis 60 °C zur vorgelegten Salpetersäure gibt.

4. Verfahren nach Anspruch 1, dadurch gekennzeichnet, daß man einen Katalysator verwendet, der Vanadat- und Kupferionen enthält.

## Claims

1. A process for the preparation of 2-methoxyalkanoic acid of 2 to 5 carbon atoms by oxidation of the corresponding 2-methoxyalkanols with nitric acid in the presence of vanadate ions as catalyst, at temperatures below 90 °C, and separation of the 2-methoxyalkanoic acid by distillation, wherein

a) less than 3 moles of nitric acid are used per mole of methoxyalkanol,

b) the oxidation is carried out at from 40 to 80 °C,

c) the reaction mixture is treated at from 40 to 100 °C with an excess of methoxyacetaldehyde or 2-methoxypropionaldehyde, and then heated to above 100 °C, while distilling off water without applying a vacuum, and

d) the residue, after isolation of the 2-methoxyalkanoic acid by fractional distillation, is used again as catalyst for the oxidation of 2-methoxyalkanol by this process.

2. A process as claimed in claim 1, wherein 1.8 to 2.5 moles of nitric acid are used per mole of 2-methoxyalkanol.

3. A process as claimed in claim 1, wherein the nitric acid and catalyst are initially charged and, prior to the addition of the 2-methoxyalkanol, from 0.05 to 2.5 % by weight, based on the aqueous nitric acid, of methoxyacetaldehyde, 2-methoxypropionaldehyde, acetaldehyde, paraldehyde, metaldehyde or propionaldehyde is added to the initially charged nitric acid at a temperature of from 40 to 60 °C.

4. A process as claimed in claim 1, wherein a catalyst is used which contains vanadate or copper ions.

## Revendications

1. Procédé de préparation d'acides 2-méthoxyalcanoïque comportant 2 à 5 atomes de carbone, par oxydation des 2-méthoxy-alcanols correspondants par de l'acide nitrique, en présence d'ions vanadates comme catalyseur, à des températures inférieures à 90 °C, en séparant par distillation l'acide 2-méthoxy-alcanoïque, caractérisé en ce que

a) l'on utilise moins de 3 moles d'acide nitrique par mole de méthoxy-alcanol,

b) on effectue l'oxydation à des températures de 40 à 80 °C,

c) on traite le mélange réactionnel à une température de 40 à 100 °C par un excès de méthoxy-acétaldéhyde ou d'aldéhyde 2-méthoxy-propionique, puis l'on chauffe en éliminant l'eau par distillation, sans faire le vide, à des températures supérieures à 100 °C, et

d) on réutilise le résidu, après isolement de l'acide 2-méthoxy-alcanoïque par distillation fractionnée, comme catalyseur pour l'oxydation de 2-méthoxy-alcanol selon le présent procédé.

2. Procédé selon la revendication 1, caractérisé en ce qu'on utilise 2,5 à 1,8 moles d'acide nitrique par mole de 2-méthoxy-alcanol.

3. Procédé selon la revendication 1, caractérisé en ce que l'on introduit préalablement l'acide nitrique et le catalyseur et avant d'ajouter le 2-méthoxy-alcanol, on ajoute 0,05 à 2,5 % en poids, par rapport à l'acide nitrique en solution aqueuse, d'aldéhyde méthoxy-acétique, d'aldéhyde 2-méthoxypropionique, d'acétaldéhyde, de paradéhyde, de métaldéhyde ou d'aldéhyde propionique, à des températures de 40 à 60 °C, à l'acide nitrique préalablement introduit.

4. Procédé selon la revendication 1, caractérisé en ce qu'on utilise un catalyseur renfermant des ions vanadate et cuivre.